# EUROPEAN PATENT APPLICATION

(11) **EP 4 494 585 A2**
(43) Date of publication of application: **22.01.2025**
(21) Application number: 24189323.9
(22) Date of filing: 18.07.2024
(51) Int. Cl.: A61B 18/12, A61B 18/14, A61B 17/00, A61B 18/00

(54) **FLEXIBLE IRRIGATION TUBE FOR A MEDICAL CATHETER**

(30) Priority: 19.07.2023 US 202363514494 P; 18.06.2024 US 202418746727
(71) Applicant: Biosense Webster (Israel) Ltd., 2066717 Yokneam (IL)
(72) Inventor: BREM, Erez Henri, 2066717 Yokneam (IL)
(74) Representative: Carpmaels & Ransford LLP

(57) **Abstract**

The disclosed technology includes an irrigation tube for a medical catheter. The irrigation tube can include a flexible tube extending along a longitudinal axis from a proximal end to a distal end. The irrigation tube can include a pull wire attached to the flexible tube, the pull wire configured to deflect at least the distal end of the irrigation tube radially outward from the longitudinal axis. The irrigation tube can include an aperture proximate the distal end of the flexible tube, the aperture configured to direct an irrigation fluid outwardly from the irrigation tube so that irrigation fluid is directed generally in a direction of deflection by the pull wire.

## Description

### CROSS REFERENCE TO RELATED APPLICATIONS

The application claims benefit of priority to prior filed U.S. Provisional Patent Application No. 63/514,494 filed July 19, 2023 (Attorney Docket No.: BIO6817USPSP1 - 253757.000352), which is hereby incorporated by reference in full herein.

### FIELD

The present invention relates generally to medical devices, and in particular medical catheters with irrigation, and further relates to, but not exclusively, medical catheters comprising a flexible irrigation tube configured to provide irrigation to electrodes.

### BACKGROUND

Cardiac arrhythmias, such as atrial fibrillation (AF), occur when regions of cardiac tissue abnormally conduct electrical signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore, cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode probes was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference and attached in the Appendix hereto.

Ablation procedures, and other procedures making use of the electrodes (e.g., electrophysiology mapping), can lead to thrombus due to the geometry of the end effector and, in some cases, localized temperature increases near the electrodes. Therefore, many existing ablation and mapping catheters include irrigation elements that are configured to deliver irrigation fluid to areas proximate the electrodes or generally at a distal end of the end effector. For example, some existing ablation catheters are configured to deliver saline to areas proximate the electrodes via an irrigation hub disposed near a proximal end of the end effector. Unfortunately, many existing irrigation elements are unable to effectively deliver irrigation fluid to the distal end of the end effector. Furthermore, many existing irrigation elements are unable to precisely direct the irrigation fluid to a selected area. Accordingly, there is a need in the art for irrigation elements that increase the effectiveness of the irrigation provided by the irrigation elements.

### SUMMARY

There is provided, in accordance with an example of the present invention, an irrigation tube for a medical catheter. The irrigation tube can include a flexible tube extending along a longitudinal axis from a proximal end to a distal end. The irrigation tube can include a pull wire attached to the flexible tube, the pull wire configured to deflect at least the distal end of the irrigation tube radially outward from the longitudinal axis. The irrigation tube can include an aperture proximate the distal end of the flexible tube, the aperture configured to direct an irrigation fluid outwardly from the irrigation tube so that irrigation fluid is generally directed in a direction of deflection by the pull wire.

The disclosed technology can include a medical probe. The medical probe can include a tubular shaft extending along a longitudinal axis. The medical probe can include a plurality of spines configured to bow radially outward from the longitudinal axis. The medical probe can include a plurality of electrodes, each electrode of the plurality of electrodes attached to a spine of the plurality of spines. The medical probe can include an irrigation tube disposed between the plurality of spines and extending distally from a distal end of the tubular shaft. At least a portion of the irrigation tube is configured to deflect radially outward from the longitudinal axis for the irrigation tube to direct an irrigation fluid to an inwardly-facing surface of an electrode of the plurality of electrodes.

Additional features, functionalities, and applications of the disclosed technology are discussed in more detail herein.

### BRIEF DESCRIPTION OF THE DRAWINGS

FIG. 1 is a schematic pictorial illustration of a medical system including a medical probe with electrodes, in accordance with the disclosed technology;
FIG. 2 is a schematic pictorial illustration showing a perspective view of a medical probe with electrodes in a deployed state, in accordance with the disclosed technology;
FIG. 3A is a schematic pictorial illustration showing a side view of a distal tip of a medical probe, in accordance with the disclosed technology;
FIG. 3B is a schematic pictorial illustration showing a top view of a distal tip of a medical probe, with spines and electrodes omitted for clarity of depiction, in accordance with the disclosed technology;
FIG. 4A is a schematic pictorial illustration showing a side view of an exemplary irrigation tube with an aperture, in accordance with the disclosed technology;
FIG. 4B is a schematic pictorial illustration showing a side view of another exemplary irrigation tube with an aperture, in accordance with the disclosed technology;
FIG. 4C is a schematic pictorial illustration showing a side view of another exemplary irrigation tube with an aperture, in accordance with the disclosed technology;
FIG. 4D is a schematic pictorial illustration showing a side view of another exemplary irrigation tube with an aperture, in accordance with the disclosed technology;
FIG. 4E is a schematic pictorial illustration showing a cross-sectional view of another irrigation tube with an aperture, in accordance with the disclosed technology;
FIG. 5A is a schematic pictorial illustration showing a side view of an exemplary irrigation tube with a resilient element and a pull string, in accordance with the disclosed technology;
FIG. 5B is a schematic pictorial illustration showing a side view of another exemplary irrigation tube with a resilient element and a pull string, in accordance with the disclosed technology;
FIG. 5C is a schematic pictorial illustration showing a side view of another exemplary irrigation tube with a resilient element and a pull string, in accordance with the disclosed technology; and
FIG. 6 is a schematic pictorial illustration showing a perspective view of a medical probe, in accordance with the disclosed technology.

### DETAILED DESCRIPTION

As discussed above, existing irrigation techniques are limited in their capabilities and, thus, oftentimes fail to achieve their intended result (e.g., cool or lubricate a specific region proximal a distal end of the medical catheter). An example of the technology that is described herein provides an irrigation tube for a medical catheter that is movable and/or steerable. In some examples, the irrigation tube includes a flexible tube extending along an axis, a steering mechanism coupled to the flexible tube and configured to deflect a distal end of the flexible tube radially outwardly and/or slide the flexible tube longitudinally, and an aperture near the distal end of the flexible tube. In some examples, the aperture is configured to direct an irrigation fluid outwardly from the irrigation tube generally to a region or location dictated by the steering mechanism. The disclosed techniques allow a physician to more precisely direct the irrigation fluid, thereby improving their ability to effectively perform an ablation or mapping procedure.

The following detailed description should be read with reference to the drawings, in which like elements in different drawings are identically numbered. The drawings, which are not necessarily to scale, depict selected examples and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as reversable or irreversible electroporation (IRE), referred throughout this disclosure interchangeably as pulsed electric field (PEF) and pulsed field ablation (PFA), or thermal energy such as radiofrequency (RF) ablation or cryoablation. Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

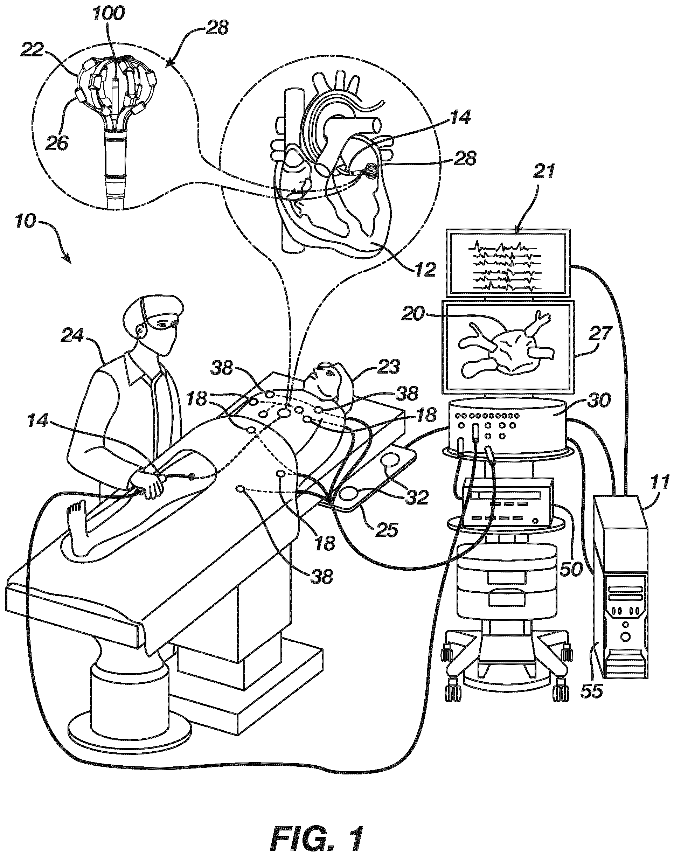

FIG. 1 shows an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a distal tip of catheter 14 (i.e., an end effector 28) into contact with the heart wall for sensing a target site in heart 12. The end effector 28 has been depicted, in this case, as a basket catheter 28; however, the end effector 28 may take other forms (e.g., it may take the form of a balloon catheter). For ablation, physician 24 similarly brings a distal end of an ablation catheter 14 to a target site for ablating.

Catheter 14 is an exemplary catheter that includes one and preferably multiple electrodes 26 optionally distributed over a plurality of spines 22 at basket catheter 28 and configured to sense the IEGM signals. Catheter 14 may additionally include a position sensor embedded in or near basket catheter 28 for tracking position and orientation of basket catheter 28. Optionally and preferably, position sensor is a magnetic based position sensor including three magnetic coils for sensing three-dimensional (3D) position and orientation. Catheter 14 can include a handle 630 at a proximal end thereof.

A combination magnetic based position sensor and force sensor may be operated together with a location pad 25 including a plurality of magnetic coils 32 configured to generate magnetic fields in a predefined working volume. Real time position of basket catheter 28 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference and attached in the Appendix hereto.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference and attached in the Appendix hereto.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

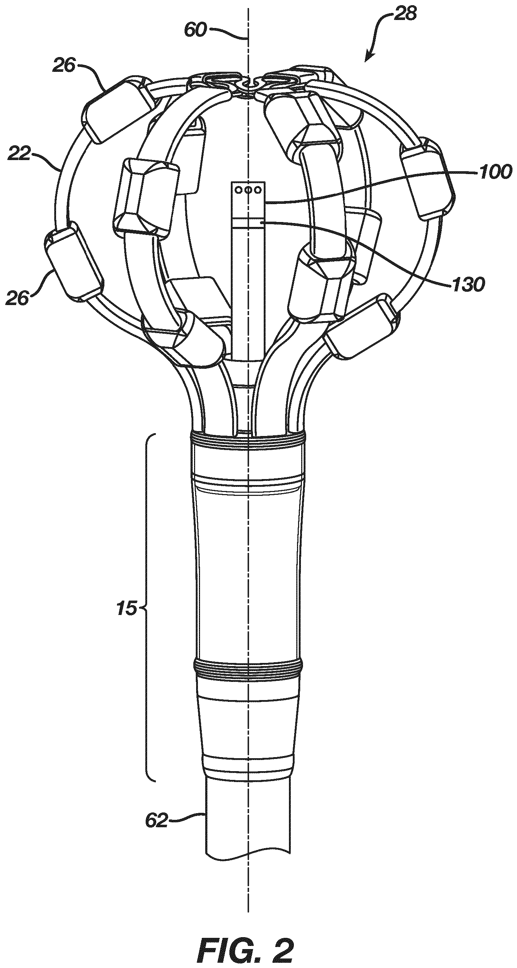

FIG. 2 is a schematic pictorial illustration showing a perspective view of a basket catheter 28 with electrodes 26 attached to spines 22 that are shown in a deployed state, such as by being advanced out of a tubular shaft lumen at a distal end of an insertion tube, in accordance with the disclosed technology. As shown in FIG. 2, the basket catheter 28 includes a plurality of flexible spines 22 that are formed at the end of a tubular shaft 62, which extends along a longitudinal axis 60, of the catheter 14 and are connected at both ends. The spines 22 can be configured to bow radially outward from the longitudinal axis 60 passing through the basket catheter 28. During a medical procedure, physician 24 can deploy basket catheter 28 by extending tubular shaft 62 from an insertion tube causing the basket catheter 28 to exit the insertion tube and transition to a deployed state from a collapsed state. In the deployed state, the spines 22 form a generally spherical shape. Spines 22 may have elliptical (e.g., circular) or rectangular (that may appear to be flat) cross-sections, and include a flexible, resilient material (e.g., a shape-memory alloy such as nickel-titanium, also known as Nitinol) forming a strut. The basket catheter 28 may be connected to a remainder of the catheter 14 by a coupler 15 positioned between tubular shaft 62 and the basket catheter 28.

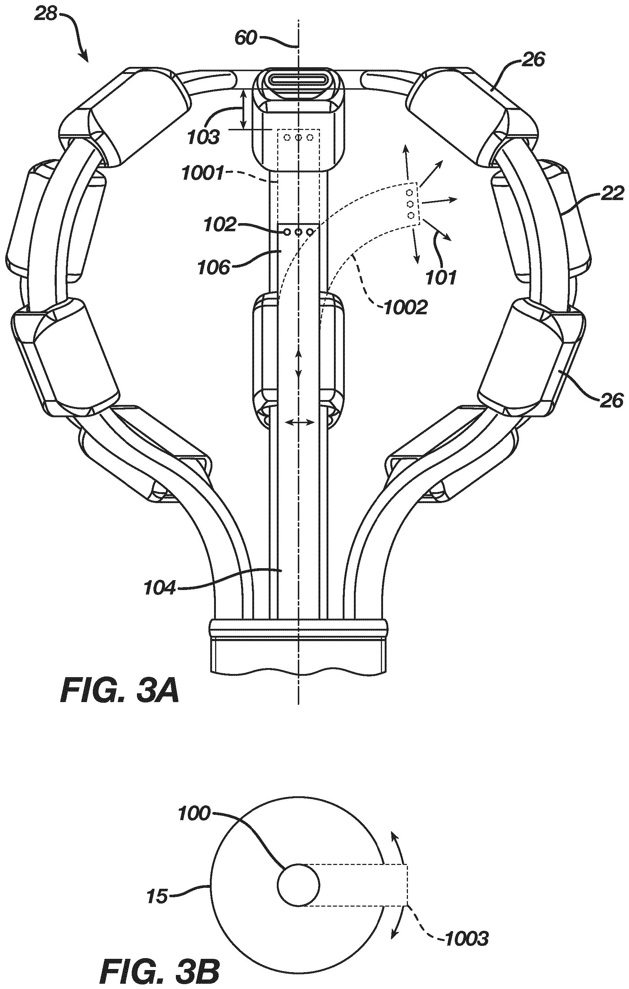

The physician 24 can bring the basket catheter 28 into contact with tissue to perform an ablation procedure and/or a mapping procedure. As ablative energy is output by the electrodes 26, the electrodes 26 and nearby tissue may begin to be heated. To help dissipate the heat generated by the electrodes 26 and/or to prevent thrombus from forming, the disclosed technology can include an irrigation tube 100 that is configured to deliver a fluid 101 (see FIG. 3A) proximate the electrodes 26 to provide a cooling effect to them, as will be described in greater detail herein. The irrigation tube 100 may include a flexible tube extending along a longitudinal axis from a proximal end 104 to a distal end 106 and, as shown in FIG. 3A, includes an aperture 102. In some examples, the irrigation tube 100 can include a flexible biocompatible material. In some examples, the aperture 102 can be configured as a single aperture. In some examples, the aperture 102 can be configured as a plurality of apertures. In some examples, the aperture 102 is defined in a sidewall of the tube 100. In some examples, the aperture 102 is defined in a distal tip of the tube 100. In some examples, a combination of the aperture 102 being defined in a sidewall and in the distal tip of the tube 100 can be employed. As shown in FIG. 2, the irrigation tube 100 extends distally from a distal end of the tubular shaft 62 and coupler 15, and it is positioned at least partially within the spherical shape of the spines 22 when they are in the deployed state.

In some examples, the irrigation tube 100 can carry one or more marker bands 130 to help the physician 24 identify the orientation of the tube 100 under fluoroscopy. Each marker band includes a metal ring of sufficient radiopacity. The marker band(s) 130 may be placed along any part of the irrigation tube 100, as long as they do not contact with any of the electrodes 26. In some examples, a connecting band used to connect pull wire(s) 108 to the tube 100 (discussed in greater detail below) may be optionally radiopaque for the same purpose.

FIG. 3A is a schematic pictorial illustration showing a side view of the basket catheter 28, the irrigation tube 100 being depicted in both solid lines and phantom lines to illustrate exemplary movement thereof. Movement of the irrigation tube 100 can be done to position the irrigation tube 100 such that the aperture 102 can direct irrigation fluid 101 to an inwardly-facing surface of a particular electrode 26 or inwardly-facing surfaces of a particular set of electrodes 26.

As shown in FIG. 3A, the irrigation tube 100 can be actuatable to slide longitudinally along the longitudinal axis 60 to a most distal position 1001 relative to an initial position thereof (solid line depiction of the irrigation tube 100 in FIG. 3A). For example, the irrigation tube 100 may slide from a proximal end of the spines 22 to a distal end of the spines 22. Alternatively, an initial position and/or an extended position of the irrigation tube 100 may be intermediate the proximal and distal ends of the spines 28. In the most distal position 1001, a gap 103 is formed along the longitudinal axis 60 between the irrigation tube 100 and the spines 22 such that the irrigation tube 100 does not contact the spines 22. In the most distal position, the gap 103 can be approximately 0.25 millimeters (mm). In other examples, the gap can be any value such as, for example, approximately 1 mm, 2 mm, 5 mm, 10 mm, etc., depending on the particular application.

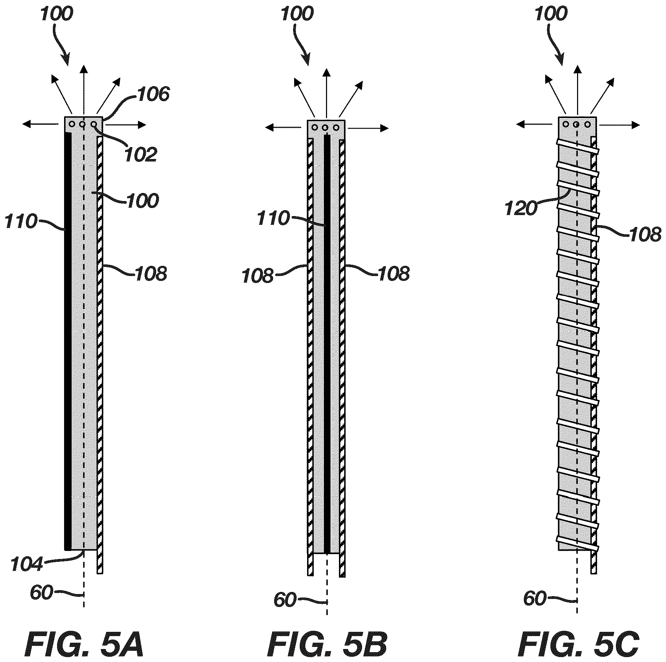

As also depicted in FIG. 3A, the irrigation tube 100 can be actuatable to deflect radially outward from the longitudinal axis 60 to a deflected position 1002 via a steering mechanism. In some examples, the steering mechanism can also or alternatively be used to actuate the tube 100 to slide longitudinally (as described in the foregoing paragraph). In some examples, the steering mechanism can include a pull wire 108 (shown in FIGs. 5A-5C) that deflects at least the distal end 106 of the irrigation tube 100 radially outward from the longitudinal axis, and is discussed in greater detail below. As an illustrative example shown in FIG. 3A, when the irrigation tube 100 is deflected to the right to the deflection position 1002, the distal end of the irrigation tube 100 faces, in a direction of deflection, towards an inwardly-facing surface of one or more electrodes 26. When the irrigation tube 100 is deflected, the aperture 102 generally directs the irrigation fluid 101 in that direction of deflection to obtain the desired cooling effect at the electrode(s) 26. Although the irrigation tube 100 is shown as being deflected to the right to the deflection position 1002, in some examples the irrigation tube 100 can be configured for deflection in any direction radially outward from the longitudinal axis.

In other examples, and as exemplified in FIG. 3B, the irrigation tube 100 can be configured for deflection in only a single direction, to a rotational position 1003, and be configured for rotation with respect to the basket assembly 28 by the physician 24 (e.g., see the directional arrows in FIG. 3B demonstrating clockwise and counterclockwise rotation). That is, the irrigation tube 100 can be deflected and then the physician 24 can rotate the irrigation tube 100 to orient the irrigation tube 100 in a desired direction. In some examples, the irrigation tube 100 can be configured deflect radially outward such that a distal end of the irrigation tube 100 maintains at least a distance of approximately 0.25 mm from an electrode 26 to prevent potential malfunctioning of the electrode 26.

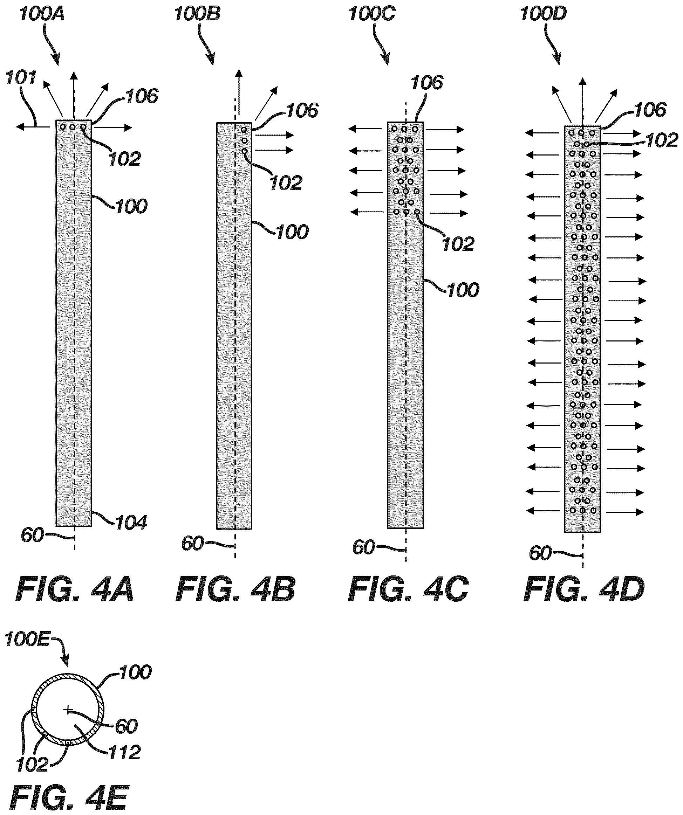

FIGS. 4A-4E are schematic pictorial illustrations showing side views of exemplary aperture configurations. As will appreciated, the aperture 102 can be configured in other ways than those depicted in FIGs. 4A-4E without departing from the spirit and scope of the disclosed technology. Furthermore, as exemplified in FIG. 4E, the irrigation tube 100 can include a lumen 112 extending at least partially therethrough that can guide irrigation fluid along a length of the irrigation tube toward the aperture 102. The lumen 112 can be sized for a desired flow rate to ensure a sufficient amount of irrigation fluid is delivered for the particular application. Similarly, the apertures 102 can be sized for a desired flow rate. In this way, a sufficient amount of fluid at a sufficient velocity can be delivered to a desired location. Furthermore, the apertures 102 can be configured to direct the fluid in a particular direction rather than just generally outward.

Making reference to example 100A in FIG. 4A, the aperture 102 can make a row along a distal edge of the distal end 106. In this example, in an undeflected position 1001, irrigation fluid 101 is primarily directed longitudinally and laterally outward from the distal end 106.

As shown in example 100B in FIG. 4B, the aperture 102 can make a column down one side of the distal end 106. In this example, a maj ority of the irrigation fluid 101 is directed to the right out of the aperture 102, which causes the irrigation tube to oscillate its position, e.g., between a first position and a second position.

As illustrated in example 100C in FIG. 4C, the aperture 102 can run along a circumference of the distal end 106 extending down a portion of the tube 100, which permits additional lateral fluid flow relative to, e.g., example 100A. The distal tip of the tube 100 can also be blocked off to further promote lateral fluid flow and prevent longitudinal flow.

Making reference to example 100D in FIG. 4D, the aperture 102 can run along a circumference of the entire tube 100, allowing fluid 101 to pass through the aperture 102 in all directions along a length of the tube 100. In this way, the irrigation tube 100 can be configured to deliver irrigation fluid along a greater length of the basket assembly 28.

FIG. 4E is a cross-sectional view (cut relative to a plane that is perpendicular to the longitudinal axis 60) of another example 100E. As shown, the aperture 102 can also be configured to cause the irrigation tube 100 to rotationally oscillate around the longitudinal axis 60 when the irrigation fluid 101 passes therethrough, similar to the depiction shown in FIG. 3B. For example, the aperture 102 can be defined in one quadrant of the tube 100 such that irrigation fluid 101 flows out the tube 100 in an inward radial direction towards the longitudinal axis 60 (i.e., to deflect the tube 100 away from the undeflected position 1001) and in a tangential direction perpendicular to the radial direction (i.e., to have a propulsion effect that rotates the tube 100 about the longitudinal axis 60).

Turning now to FIGS. 5A-5C, schematic pictorial illustrations showing side views of exemplary irrigation tube 100 configurations are presented. In these figures, various examples of pull wire 108 and resilient element 110 arrangements are used to, respectively, steer the irrigation tube 100 to deflect at least the distal end 106 radially outward from the longitudinal axis 60 and return the irrigation tube 100 to a position aligned with the longitudinal axis 60. The pull wire 108 and the resilient element 110 can be made from a biocompatible material such a Nitinol, cobalt chromium, titanium, stainless steel, biocompatible polymers, or other suitable materials.

In some examples, the pull wire 108 runs from an inner portion of the distal end 106, to which an end of the pull wire is coupled (e.g., via a connecting band), along an inner wall of the tube 100 and out through the proximal end 104 to connect with a deflection drive actuator 632 (described in greater detail below). In some examples, the pull wire may be coupled to the distal end 106 on an outer wall thereof. In some examples, an adhesive may be used to connect the pull wire 108 to the tube 100. In some examples, a mechanical process may be used to connect them. However, any appropriate positioning arrangement and coupling method may be employed in accordance with the disclosed technology.

In some examples, the resilient element 110 runs from the distal end 106 and terminates at the proximal end 104 of the tube 100. The resilient element 110 can be attached to or embedded in any appropriate location of the tube 100. In some examples, the resilient element 110 can include an elongated rod. In some examples, the resilient element 110 can include a spring 120, such as a coil spring wrapped around the tube 100 (as shown in FIG. 5C). In some examples, the tube 100 itself can be made from a resilient material such that the irrigation tube 100 can return to a position aligned with the longitudinal axis 60.

As shown in FIG. 5A, a pull wire 108 can be run along one side of the tube 100. In this example, when the pull wire 108 is pulled, depending upon where it is mounted to the distal end 106, the distal end is either radially directed left or right (e.g., see the deflected position 1002 in FIG. 3A) outward from the longitudinal axis 60. To bias the tube 100 back to its original position, the resilient element 110 can be attached to or embedded within the tube 100, as mentioned above. In the illustrated example, the element 110 is opposite the pull wire 108, but other locations are possible. The pull wire 108 and resilient element 110 work in conjunction to ensure precise positioning of the distal end 106 such that the irrigation fluid 101 can be delivered to the intended location or region.

FIG. 5B shows another example of a configuration of pull wire 108 and resilient element 110. In this example, two pull wires 108 are coupled on opposing sides of the tube 100, with the resilient element 110 secured to the tube 100 between the wires 108. With this arrangement, pulling one of the pull wires 108 causes the distal end 106 to turn in a particular direction. By way of example, the left pull wire 108 can be coupled to the tube 100 so as to cause the distal end 106 to tilt to the left when pulled, and the right pull wire 108 can be coupled to the tube 100 so as to cause the distal end 106 to tilt to the right when pulled. Thus, multiple pull wires 108 can facilitate more customized placement of the distal end 106 when in use.

FIG. 5C shows yet another example of tube 100 with a pull wire 108 and a resilient element 110. This example is similar to that of the one shown in FIG. 5A, with the difference being that a coil spring 120 is used as the resilient element 110. While functionally similar to the previously described examples, as those skilled in the art will appreciate, different structural elements can provide their own unique functional advantages. For example, using a coil spring 120 that extends around the tube 100, the pull wire 108 can be routed through the coil spring 120. This ensures the pull wire 108 is held against the tube 100 and cannot be extended away from it, even when the pull wire 10 is pulled.

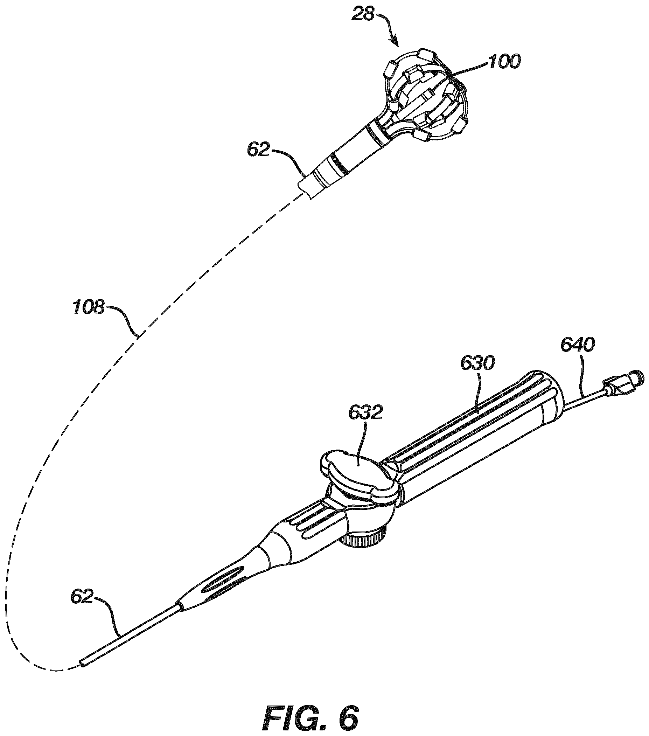

FIG. 6 is a schematic pictorial illustration of a perspective view of the catheter 14, with an intermediate portion of the shaft 62 cut away for illustrative purposes. Moreover, as will be appreciated, components are not necessarily shown to scale, for clarity. As shown, the handle 630 is connected to the tubular shaft 62 at a proximal end thereof. The handle 630 can include a deflection drive actuator 632 and an irrigation fluid conduit 640. The conduit 640 is in fluidic communication with the irrigation tube 100 to route irrigation fluid 101 thereto, as controlled by the physician 24. The pull wire 108, which extends through the tubular shaft 62, has a proximal end connected to the deflection drive actuator 632. Like the pull wire's connection to the tube 100, any appropriate coupling technique may be employed. When the deflection drive actuator 632 is actuated (i.e., by the physician 24), the pull wire 108 can be pulled and the irrigation tube 100 deflected radially outward from the longitudinal axis 60. If more than one pull wire 108 is attached to the irrigation tube 100, actuating the deflection drive actuator 632 in a first direction can cause the irrigation tube 100 to deflect in a first direction while actuating the deflection drive actuator 632 in a second direction can cause the irrigation tube 100 to deflect in a second direction. In some examples, the deflection drive actuator can be twisted to wrap the proximal end of the pull wire 108 therearound, thereby causing the actuation of the pull wire that steers and/or deflects the irrigation tube 100 in a manner as described above. However, other forms of actuation may be employed, such as, but not limited to, a slider that selectively pulls and pushes the pull wire 108 to effect deflection of the tube 100.

The disclosed technology described herein can be further understood according to the following clauses:
Clause 1: An irrigation tube for a medical catheter, the irrigation tube comprising: a flexible tube extending along a longitudinal axis from a proximal end to a distal end; a pull wire attached to the flexible tube, the pull wire configured to deflect at least the distal end of the flexible tube radially outward from the longitudinal axis; and an aperture proximate the distal end of the flexible tube, the aperture configured to direct an irrigation fluid outwardly from the irrigation tube so that irrigation fluid is generally directed in a direction of deflection by the pull wire.
Clause 2: The irrigation tube of Clause 1, the irrigation tube comprising a flexible biocompatible material.
Clause 3: The irrigation tube of Clause 1 or Clause 2, the pull wire configured to be attached to a handle of the ablation catheter, the handle comprising a deflection drive actuator connected to a proximal end of the pull wire, the deflection drive actuator configured to actuate the pull wire to deflect the irrigation tube.
Clause 4: The irrigation tube of any one of Clauses 1-3, further comprising a resilient element configured to return the irrigation tube to a position aligned with the longitudinal axis.
Clause 5: The irrigation tube of Clause 4, the resilient element comprising an elongated rod attached to the irrigation tube.
Clause 6: The irrigation tube of Clause 4, the resilient element comprising a spring attached to the irrigation tube.
Clause 7: The irrigation tube of any one of Clauses 1-3, wherein the irrigation tube is made from a resilient material such that the irrigation tube is configured to return to a position aligned with the longitudinal axis.
Clause 8: The irrigation tube of any one of Clauses 1-7, wherein the irrigation tube is further configured to slide longitudinally with respect to a plurality of spines of the ablation catheter from a proximal end of the plurality of spines to a distal end of the plurality of spines.
Clause 9: The irrigation tube of any one of Clauses 1-8, the aperture being configured to direct the irrigation fluid toward an electrode.
Clause 10: The irrigation tube of any one of Clauses 1-9, the aperture configured to cause the irrigation tube to oscillate between a first position and a second position when the irrigation fluid passes therethrough.
Clause 11: The irrigation tube of any one of Clauses 1-9, the aperture configured to cause the irrigation tube to rotationally oscillate around the longitudinal axis when the irrigation fluid passes therethrough.
Clause 12: A medical probe, comprising: a tubular shaft extending along a longitudinal axis; a plurality of spines configured to bow radially outward from the longitudinal axis; a plurality of electrodes, each electrode of the plurality of electrodes attached to a spine of the plurality of spines; and an irrigation tube disposed between the plurality of spines and extending distally from a distal end of the tubular shaft, at least a portion of the irrigation tube configured to deflect radially outward from the longitudinal axis for the irrigation tube to direct an irrigation fluid to an inwardly-facing surface of an electrode of the plurality of electrodes.
Clause 13: The medical probe of Clause 12, the irrigation tube comprising a flexible biocompatible material.
Clause 14: The medical probe of Clause 12 or Clause 14, the irrigation tube being steerable such that a distal end of the irrigation tube can be moved to direct the irrigation fluid toward the electrode of the plurality of electrodes.
Clause 15: The medical probe of any one of Clauses 12-14, further comprising a pull wire attached to the irrigation tube, the pull wire configured to steer the irrigation tube.
Clause 16: The medical probe of Clause 15, further comprising a handle attached to a proximal end of the tubular shaft, the handle further comprising a deflection drive actuator connected to a proximal end of the pull wire, the deflection drive actuator configured to actuate the pull wire to steer the irrigation tube.
Clause 17: The medical probe of any one of Clauses 12-16 further comprising a resilient element configured to cause the irrigation tube to return to a position aligned with the longitudinal axis.
Clause 18: The medical probe of Clause 17, the resilient element comprising an elongated rod attached to the irrigation tube.
Clause 19: The medical probe of Clause 17, the resilient element comprising a spring attached to the irrigation tube.
Clause 20: The medical probe of any one of Clauses 12-19, wherein the irrigation tube is further configured to slide longitudinally along the longitudinal axis from a proximal end of the plurality of spines to a distal end of the plurality of spines.
Clause 21: The medical probe of Clause 20, wherein, in a most distal position of the irrigation tube, a gap is formed between the irrigation tube and the plurality of spines such that the irrigation tube does not contact the plurality of spines.
Clause 22: The medical probe of Clause 21, the gap being approximately 0.25 mm.
Clause 23: The medical probe of any one of Clauses 12-16 and 20-22, wherein the irrigation tube is made from a resilient material such that the irrigation tube is configured to return to a position aligned with the longitudinal axis.
Clause 24: The medical probe of any one of Clauses 12-23, the irrigation tube comprising an aperture configured to direct the irrigation fluid toward the electrode of the plurality of electrodes.
Clause 25: The medical probe of Clause 24, the aperture configured to cause the irrigation tube to oscillate between a first position and a second position when the irrigation fluid passes therethrough.
Clause 26: The medical probe of Clause 24, the aperture configured to cause the irrigation tube to rotationally oscillate around the longitudinal axis when the irrigation fluid passes therethrough.
Clause 27: The medical probe of any one of Clauses 12-26, the irrigation tube being configured deflect radially outward such that a distal end of the irrigation tube maintains at least a distance of approximately 0.25 mm from an electrode.
Clause 28: The medical probe of any one of Clauses 12-27, the plurality of spines being configured to transition between a collapsed state and a deployed state.
Clause 29: The medical probe of Clause 28, the plurality of spines forming a generally spherical shape when in the deployed state.
Clause 30: The medical probe of Clause 29, the irrigation tube being positioned at least partially within the spherical shape when the plurality of spines are in the deployed state.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. An irrigation tube for a medical catheter, the irrigation tube comprising:
a flexible tube extending along a longitudinal axis from a proximal end to a distal end;
a pull wire attached to the flexible tube, the pull wire configured to deflect at least the distal end of the flexible tube radially outward from the longitudinal axis; and
an aperture proximate the distal end of the flexible tube, the aperture configured to direct an irrigation fluid outwardly from the irrigation tube so that irrigation fluid is generally directed in a direction of deflection by the pull wire.

2. The irrigation tube of claim 1, further comprising a resilient element configured to return the irrigation tube to a position aligned with the longitudinal axis.

3. The irrigation tube of claim 2, the resilient element comprising i) an elongated rod attached to the irrigation tube, or ii) a spring attached to the irrigation tube.

4. The irrigation tube of claim 1 or claim 2, wherein the irrigation tube is made from a resilient material such that the irrigation tube is configured to return to a position aligned with the longitudinal axis.

5. The irrigation tube of any preceding claim, the aperture being configured to direct the irrigation fluid toward an electrode.

6. The irrigation tube of any preceding claim, the aperture configured to cause the irrigation tube i) to oscillate between a first position and a second position when the irrigation fluid passes therethrough, or ii) to rotationally oscillate around the longitudinal axis when the irrigation fluid passes therethrough.

7. A medical probe, comprising:
a tubular shaft extending along a longitudinal axis;
a plurality of spines configured to bow radially outward from the longitudinal axis;
a plurality of electrodes, each electrode of the plurality of electrodes attached to a spine of the plurality of spines; and
an irrigation tube disposed between the plurality of spines and extending distally from a distal end of the tubular shaft, at least a portion of the irrigation tube configured to deflect radially outward from the longitudinal axis for the irrigation tube to direct an irrigation fluid to an inwardly-facing surface of an electrode of the plurality of electrodes.

8. The medical probe of claim 7, the irrigation tube being steerable such that a distal end of the irrigation tube can be moved to direct the irrigation fluid toward the electrode of the plurality of electrodes.

9. The medical probe of claim 7, or claim 8 further comprising a pull wire attached to the irrigation tube, the pull wire configured to steer the irrigation tube, optionally further comprising a handle attached to a proximal end of the tubular shaft, the handle further comprising a deflection drive actuator connected to a proximal end of the pull wire, the deflection drive actuator configured to actuate the pull wire to steer the irrigation tube.

10. The medical probe of any of claims 7 to 9, further comprising a resilient element configured to cause the irrigation tube to return to a position aligned with the longitudinal axis.

11. The medical probe of any of claims 7 to 10, wherein the irrigation tube is further configured to slide longitudinally along the longitudinal axis from a proximal end of the plurality of spines to a distal end of the plurality of spines, optionally wherein, in a most distal position of the irrigation tube, a gap is formed between the irrigation tube and the plurality of spines such that the irrigation tube does not contact the plurality of spines.

12. The medical probe of claim 7, wherein the irrigation tube is made from a resilient material such that the irrigation tube is configured to return to a position aligned with the longitudinal axis.

13. The medical probe of claim 7, the irrigation tube comprising an aperture configured to direct the irrigation fluid toward the electrode of the plurality of electrodes.

14. The medical probe of claim 13, the aperture configured to cause the irrigation tube i) to oscillate between a first position and a second position when the irrigation fluid passes therethrough, or ii) to rotationally oscillate around the longitudinal axis when the irrigation fluid passes therethrough.

15. The medical probe of claim 9, the irrigation tube being configured deflect radially outward such that a distal end of the irrigation tube maintains at least a distance of approximately 0.25 mm from an electrode.
